(19) Europäisches Patentamt / European Patent Office / Office européen des brevets

(11) **EP 4 398 822 B1**

(12) **EUROPEAN PATENT SPECIFICATION**

(45) Date of publication and mention
of the grant of the patent:
**04.02.2026  Bulletin 2026/06**

(21) Application number: **22783369.6**

(22) Date of filing: **06.09.2022**

(51) International Patent Classification (IPC):
**A61B 18/12** *(2006.01)*     **A61B 17/32** *(2006.01)*
**A61B 18/14** *(2006.01)*     **A61B 17/3203** *(2006.01)*
**A61B 18/00** *(2006.01)*

(52) Cooperative Patent Classification (CPC):
**A61B 17/3203; A61B 17/320068; A61B 18/12;**
**A61B 18/148;** A61B 2017/320078;
A61B 2017/32035; A61B 2018/00029;
A61B 2018/00589; A61B 2018/00601;
A61B 2018/00994; A61B 2018/1253;
A61B 2018/142; A61B 2217/007; A61B 2218/005

(86) International application number:
**PCT/IB2022/058358**

(87) International publication number:
**WO 2023/031893 (09.03.2023 Gazette 2023/10)**

(54) **SURGICAL DEVICE FOR DISSECTION OF ANATOMICAL TISSUES**

CHIRURGISCHE VORRICHTUNG ZUR DISSEKTION VON ANATOMISCHEN GEWEBEN

DISPOSITIF CHIRURGICAL POUR LA DISSECTION DE TISSUS ANATOMIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Validation States:
**MA TN**

(30) Priority: **06.09.2021  IT 202100022994**

(43) Date of publication of application:
**17.07.2024  Bulletin 2024/29**

(73) Proprietor: **Rescue Code S.r.l.
73024 Maglie (LE) (IT)**

(72) Inventor: **MASSETTI, Massimo
73024 Maglie (LE) (IT)**

(74) Representative: **Pace Napoleone, Maria et al
De Simone & Partners S.r.l.
Via Giulio Caccini, 1
00198 Roma (IT)**

(56) References cited:
WO-A2-03/096871     US-A1- 2019 125 455
US-A1- 2020 093 537

## Description

### TECHNICAL FIELD

The present invention refers to a device for surgical applications suitable for dissecting anatomical tissues.

### PRIOR ART

The interest in the two techniques of hydro-dissection and electro-dissection in the field of surgery is relatively recent. These are specialised techniques used to perform tissue dissection with remarkable precision during specific surgical procedures.

More in detail, hydro-dissection involves using a jet of liquid emitted at a pressure that induces the separation of the incident tissues with lower resistance. The application of this technique in cataract surgery has proven its effectiveness and efficiency with the result that its use has been known for years.

Electro-dissection typically refers to a monopolar dissection technique that uses electrical energy to separate tissues through the sublimation process. This technique is used in all surgeries and allows the dissection and the cauterization of the tissues while ensuring haemostasis.

Although both are generally effective, these dissection technologies present criticalities that unfortunately limit the use thereof in some surgical situations. Typically, the use of these technologies requires first of all the use of a handpiece (scalpel) that has the shape of a pencil with a thin tip. This tip allows the mechanical separation of the tissues with the jet of the liquid in hydro-dissection and with the heat in dissection promoted by the passage of monopolar current. During electro-dissection, tissue separation and haemostasis are caused by the passage of electrical current through the anatomical tissue. Obviously, the greater the potency it is, the greater the risk of unwanted thermal necrosis in nearby tissues (thermal damage that causes tissue necrosis). In a monopolar diathermy procedure, an electrical current flows from the plate of any random electrode (usually attached to the patient's thigh) to the blade (or tip) of the scalpel manipulated by the surgeon. The current flows through the body creating thermal damages in the tissues near the blade of the scalpel with consequent clotting of the tissues.

However, undesirable consequences may occur, the main ones being thermal damages to the adjacent tissue and the production of smoke due to the sublimation of the tissue itself. These damages are especially frequent when operating on anatomical tissues adjacent to vascular structures, like in cardiovascular surgery. Similar damages may occur during other surgeries in which the respect of the structural integrity of the tissues adjacent to the site of use of the scalpel is required. It should also be noted that the accumulation of eschar, which inevitably occurs on the stainless steel tip of the handpiece, increases the electrical resistance creating the need for the electrical power to be raised. It is noted that an increase in power in addition to making electrosurgery less efficient, can also potentially be more dangerous due to the smoke that is produced with the tissue charring. It should also be pointed out that in the electrosurgical smoke there are blood, viral particles, DNA, bacteria, water, carbon dioxide and toxic gases. These airborne particles are small enough to penetrate deep into the respiratory tract and are considered potentially hazardous to medical personnel who is surgically exposed.

Smoke evacuation has therefore become an important issue regulated by legislation on the safety of health workers in the workplace. In short, the scalpel becomes potentially dangerous if, when used, tissues are burned causing necrosis thereof.

The presence of this danger has the effect of requiring the attention of the surgeon, who performs the operations taking much more time than he would have done if this problem did not exist.

The scalpel itself is based on a "monopolar" electrode. In essence, the electrical current passes through this electrode and the patient's body through a second pole hooked to the patient's body. What is indicated by the expression "burning of a tissue" that causes the unwanted necrosis thereof is actually a phenomenon that can be summarized in two distinct steps:

- the tissue loses its water content locally; and
- the energy that is transferred to the tissue, locally dissected, causes the "combustion" thereof, and hence the necrosis.

The produced heat develops due to the Joule effect.

It is evident that the "dissection" of the tissue in contact with the arc, which precedes its "combustion" and hence the necrosis, causes an increase in the electrical resistance of the tissue itself. The water that the tissue contains is in fact a good conductor.

Furthermore, it can be noted that, during the phase preceding the undesired necrosis, in the circuit consisting of the patient's body and in the electrical current coming from the electrical source that powers the scalpel, there is a progressive decrease in voltage.

In fact, according to the Ohm's law:

$$current = voltage /electrical resistance$$

The increase of the applied electrical resistance leads to a decrease in the current, which makes sense also from a thermodynamic point of view.

It is therefore possible to measure the electrical parameters of the circuit and determine the maximum tolerable increase, building correlations existing between data and types of the tissue.

Furthermore, the light spectrum of the arc is modified both as a function of the tissue subject to the action and as a function of the process of "degradation" towards necrosis. The arc light can be easily detected by a sensor, made with a diffraction grating or a photodiode array through an optical fibre. A detection method that is obtained by positioning the sensor very "close" to the arc while maintaining dimensions such as to make the presence thereof "imperceptible".

In cardiac surgery, coronary revascularization is carried out by creating bypasses, using conduits (arteries and veins) that are taken from the same patient. Currently, the internal thoracic artery (ITA) is considered the conduit of choice for its high rate of long-term permeability. For preparing it, during the procedure, use is made of the monopolar scalpel which allows the separation of the artery from the surrounding tissues and from the collateral vessels. The quality of the preparation of the conduit and the results of this surgery also depend on the appropriate use of the handpiece and on the risk of thermal damage to the wall when operating near the vessel. Regardless of the technique, the damage to the artery, particularly to the endothelium, is considered an event that corresponds to an important risk factor for early or late graft failure. For this reason, new technologies have been proposed to allow the improvement of the technique for the preparation of the conduits in surgery.

Therefore, it is an object of the present invention to provide a surgical device that partially or totally overcomes the drawbacks of the known devices. In particular, it is an object of the present invention to realize a surgical device that allows to perform, in the context of a surgical procedure, the dissection of the anatomical tissues not only with high precision but above all avoiding that the aforementioned disadvantageous consequences of the dissection that are correlated, for example, to the production of smoke and/or to the undesired necrosis of the adjacent tissues occur. The whole with the result of making the surgical procedure highly improved both in terms of the effectiveness thereof and the postoperative duration thereof.

Specifically, it is an object of the present invention to provide a surgical device that allows a rapid dissection with the ability of separating the tissues with lower resistance, which allows haemostasis of the vessels avoiding excessive bleeding, which allows a diathermy without tissue charring and production of toxic smoke and which can be used in different surgical applications.

US2020/093537A1 discloses an HF-surgical preparation instrument. WO03/096871A2 discloses a multi-purpose surgical instrument which uses high-pressure liquid jets and an electrode.

## DESCRIPTION OF THE INVENTION

These objects are achieved by a surgical device according to the claims at the end of the present disclosure.

In one aspect of the invention, a surgical device for dissecting anatomical tissue is provided. The device comprises a main body extending along a longitudinal direction and having a first end and a second end comprising a contact portion adapted to come into contact with the anatomical tissue, in particular having a spatula shape, wherein the contact portion comprises a first sub-portion and a second sub-portion extending over the first sub-portion. The device further comprises at least a first dissecting means positioned on the first sub-portion and at least a second dissecting means positioned on the second sub-portion, wherein the first dissecting means is disposed on an end region of the second end and the second dissecting means is retracted and spaced from the first dissecting means along the longitudinal direction of the main body at a distance d. The device further comprises a fluid dispensing means positioned on the second sub-portion and side-by-side to the second dissecting means, said fluid dispensing means being connectable to a fluid reservoir through a connector at the first end.

In this way, a cutting device is obtained with which the dissection of the anatomical tissues can be performed with high precision avoiding all the criticalities associated with the pre-existing technologies.

It is noted that the particular configuration of the two dissecting means, i.e. the arrangement of the second dissecting means retracted with respect to the first dissecting means increases the possibility of performing the dissection of the tissues of interest with accurate precision. In fact, the two dissecting means can use different technologies and can be used for dissecting more or less resistant tissues. For example, the first dissecting means may be employed for dissecting more resistant tissues while the second dissecting means may be employed for dissecting less resistant tissues. The staggered position along the longitudinal direction of the main body between the two dissecting means allows the two means to be used without the risk of an interference in the implementation of either means. In particular, the use of the first dissecting means may take place simultaneously with the use of the second dissecting means.

The presence of the fluid dispensing means makes it possible to clean the field from bleeding during the dissection operation and to identify the bleeding source. In addition, the fact that the fluid dispensing means is positioned on the second sub-portion and is side-by-side to the second dissecting means serves to ensure a greater effectiveness of the dissection action of the device.

This and other aspects of the present invention will become clearer in the light of the following description of some preferred embodiments described below.

Fig. 1 shows a schematic representation of the device according to an example.

Fig. 2A-C show a schematic representation of a surgical device according to another example.

Fig. 3A-C show a schematic representation of a surgical device according to a further example.

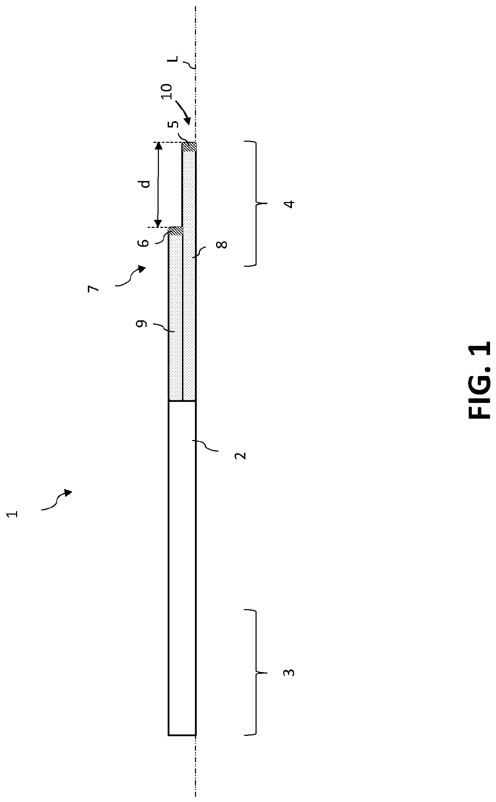

Figure 1 shows in a schematic representation a side section of the surgical device 1 for dissecting anatomical tissues.

The surgical device 1 can be understood as a surgical scalpel or more generally as a cutting device. The device 1 comprises a main body 2 or handpiece adapted to be manoeuvred by the specialist surgeon. The main body 2 may have a cylindrical profile but may also assume other advantageous forms and extends along a longitudinal direction L. The body 2 comprises a first end 3 and a second end 4 comprising a contact portion 7 adapted to come into contact with the anatomical tissue. Specifically, the contact portion 7 can be suitably manoeuvred to allow the surgeon to perform the actual dissection of the anatomical tissues of interest.

In particular, the contact portion 7 may advantageously have a spatula shape and comprises a first sub-portion 8 and a second sub-portion 9 extending over the first sub-portion 8. These two sub-portions essentially represent two layers of the contact portion 7, in which the second sub-portion 9 is positioned over the first sub-portion 8 and is shorter than the latter. In other words, the first sub-portion 8 extends as far as the end region 10 of the device 1 at the second end 4, whereas the second sub-portion 9 is shorter and does not extend as far as said end region 10.

The device 1 comprises a first dissecting means 5 positioned on the first sub-portion 8 disposed on the end region 10 of the second end 4 and a second dissecting means 6 positioned on the second sub-portion 9. Due to the different length of the two sub-portions, the second dissecting means 6 is retracted and spaced from the first dissecting means 5 along the longitudinal direction L of the main body 2 at a distance d.

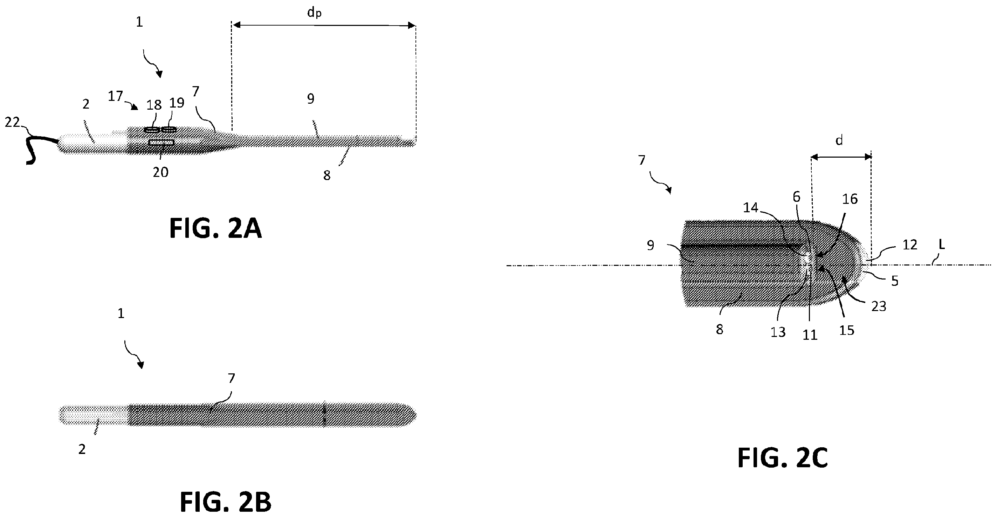

Figures 2A-2C show the surgical device 1 according to an embodiment. In detail, figure 2A shows a side view of the device 1 while figure 2B shows a top view. From these figures it can be appreciated that the contact portion 7 is connected to the main body 2 and assumes a spatula shape with a pointed termination. This configuration is particularly advantageous for operating tissue dissection with precision. The first and second end 3, 4 may both have a truncated conical profile. The contact portion 7 is, at least partially, deformable and malleable in order to be able to easily fit between the folds of the anatomical tissues. Furthermore, the contact portion can be detached and attached, i.e. removably attachable, to the main body 2 at the second end 4. Figure 2C shows a detail of the tip of the contact portion 7.

In the example, the device 1 comprises a fluid dispensing means 11 positioned on the second sub-portion 9 near the second dissecting means 6. This can be appreciated in figure 2C. The fluid dispensing means 11 is side-by-side to the second dissecting means 6 on the second sub-portion 9. The fluid dispensing means 11 is advantageously connectable to a fluid reservoir through a connector at the first end 3. In this context, the term fluid refers to a gas, a liquid, or a mixture of gas and liquid. The fluid dispensing means 11 comprises at least a first nozzle 13 for dispensing a pressurized fluid, in particular for dispensing carbon dioxide. Of course, other appropriate gases or mixtures of gases and liquids may be employed for the same purpose. The fluid flow (for example $CO_2$) when exiting the first nozzle 13, allows to clean the field from bleeding during the dissection operation and to identify the bleeding source.

The second dissecting means 6 comprises a second nozzle 14 for dispensing a pressurized fluid, in particular for dispensing a saline solution. Of course, other appropriate fluids can be employed for the same purpose. In other words, this second dissecting means serves for a hydro-dissection of the anatomical tissue. In particular, the second dissecting means 6 is connectable to a fluid reservoir through a connector at the first end 3.

From figure 2C it can be noted that the first nozzle 13 is side-by-side to the second nozzle 14 and that the first nozzle 13 and the second nozzle 14 respectively comprise a first outlet orifice 15 and a second outlet orifice 16. The fact that the first nozzle 13 is side-by-side to the second nozzle 14 serves to ensure that the two flows exiting the corresponding nozzles (the fluid jet (e.g. gas) exiting the fluid dispensing means 11 and the fluid jet (e.g. liquid) exiting the second dissecting means 6) converge into a single region in front of the first dissecting means 5. According to the configuration of figure 2C, the first outlet orifice 15 and the second outlet orifice 16 are arranged on the same plane, orthogonal to the longitudinal direction L of the main body 2. In other words, the fluid jet (e.g. gas) exiting the fluid dispensing means 11 and the fluid jet (e.g. liquid) exiting the second dissecting means 6 exit two adjacent points along the main body 2, i.e. along the contact portion 7 of the device 1.

The first dissecting means 5 comprises an electrode 12 for an electro-dissection of the anatomical tissue. In particular, the electrode 12 is connectable to a current generator via a connector at the first end 3. Advantageously, the connectors for the first dissecting means 5, for the second dissecting means 6 and for the fluid dispensing means 11 can be passed within a single sheath cable 22 fixed to the first end 3 of the device 1.

Alternatively or additionally, the first dissecting means 5 may comprise an ultrasound emitter for dissection by ultrasound induction of the anatomical tissue. The emitter is connectable to a current generator through a connector at the first end 3.

Thanks to a synergistic combination of structural elements present therein that are respectively typical of the instrumentation relating to the hydro-dissection and to electro-dissection of anatomical tissues, the device 1 is extremely effective in precisely separating both the tissues with lower resistance and those with greater resistance. Furthermore, the device 1 allows a timely identification of any bleeding sources and guarantees the early haemostasis of the vessels avoiding the occurrence of relatively excessive bleeding. Advantageously, the device 1 also performs diathermy without charring the tissues adjacent to the dissected ones and the undesired production of toxic smoke that is harmful especially to the medical personnel involved in the implementation of the surgical procedure. In addition, the device 1 is such as to be applicable in all types of surgical procedures and not limited to areas of medicine in which hydro-dissection or electro-dissection technologies are typically used.

Specifically, the device 1 represents a hybrid system comprising at least a first dissecting means 5 to favour diathermy, typically, but not necessarily, induced by monopolar current. The device 1 can in turn comprise at least one orientable blade so as to be adaptable to the specific type of surgical application. Furthermore, the device comprises a second dissecting means 6 for a hydro-dissection that involves emitting a spray jet of a fluid comprising saline solution. In addition, the device 1 comprises a fluid dispensing means (e.g. $CO_2$) 11. Attention is drawn to the fact that the traditional dissection typically requires the presence of additional personnel who improve the exposure of the tissues to be dissected and simultaneously aspirate the bleeding that inevitably arises following the separation of the tissues. With this device 1, the presence of additional personnel is no longer necessary. Taking into account these criticalities, the device 1 of figure 2A-2C comprises a hybrid system that combines the structural components for inducing both diathermy and hydro-dissection as well as for the early removal of any toxic smokes produced during the surgical procedure. This device 1 thus makes it possible to achieve a dissection of the tissues that is decidedly faster, more precise and safer than the known technology.

More in detail, the device 1 according to this example provides that a jet of pressurized saline solution outflows from at least one second nozzle 14 so as to perform the separation of the anatomical tissues with lower resistance, also creating a micro oedema of the soft tissues and that a jet of $CO_2$ fluid outflows from at least one first nozzle 13 (typically coplanar with that from which the saline jet outflows) to perform the cleaning of the area involved in the dissection and in the bleeding and also to identify the source itself of the bleeding which is then coagulated by diathermy induced, for example, by the passage of monopolar current. It is noted that the fluid jet dispensed by the second nozzle 14 - also in combination with the fluid jet dispensed by the first nozzle 13 - can be advantageously employed to cool the coagulation site by limiting the heat damage on the adjacent structures and avoiding charring the tissues with the production of toxic smoke.

Even more in detail, the jet of $CO_2$ fluid is dispensed continuously from the first nozzle 13. Furthermore, the device comprises a flow regulator 20 disposed on the main body 2 for dosing the amount of fluid flow exiting the first nozzle 13. In particular, the fluid flow is variable between 0 l/min and 1 l/min.

The device 1 according to this example, comprises an actuation portion 17 arranged on the main body 2 and having a first button 18 for activating the first dissecting means 5 and a second button 19 for activating the second dissecting means 6. In this way, during the dissection procedure, the operator therefore has the possibility to alternately use, through said specific dispensing buttons, the hydro-dissection jet for separating the tissues with lower resistance and the electrocoagulation for favouring haemostasis and for cutting the structures with greater mechanical resistance such as the vessels through an electro-dissection. As already highlighted, the saline solution, projected under pressure and emitted by the second nozzle 14 also allows to promote the formation of an artificial oedema with the aim of better delimiting the planes of the tissues according to their mechanical resistance and it also provides a continuous cooling of the tissue during cauterization thus reducing the potential for thermal damages and the production of toxic smoke.

From figure 2C it can be seen that not only the second dissecting means 6 is placed at a distance d from the first dissecting means 5 but also the fluid dispensing means 11 is similarly spaced from the first dissecting means 5. Furthermore, it can be noted that the first sub-portion 8 is shaped like a strip and has an arcuate end 23 where an electrode 12 is present while the second sub-portion 9 rises like a relief, at least partially, on the surface of the first sub-portion 8. The second sub-portion 9 is also shaped like a strip and has a smaller extension than the first sub-portion 8 shaped like a strip, both in terms of length and width and is spatially configured as a relief that rises on at least one of the two surfaces of the first sub-portion 8. Unlike the latter, the second sub-portion 9 does not have any arcuate end but rather a linear one, that is, it has a straight end to be directed towards the tissue to be dissected.

As already described, at the straight end of the second sub-portion 9 there is, in addition to the first nozzle 13, also the second nozzle 14 for the emission of a saline solution in the form of an aerosol to perform the hydro-dissection of the anatomical tissues that are to be separated and/or for the definition of an artificial oedema adapted to delimit the area that the surgeon must treat. The first nozzle 13 is coplanar with the second nozzle 14 and adapted to emit $CO_2$ gas that plays the dual role of removing any production of toxic smoke produced following the dissection promoted by the diathermy typically induced by the monopolar current, and also of identifying early the source of any bleeding arising following said dissection. All this making the cauterization process faster and more effective.

The first sub-portion 8, and more precisely the arcuate end thereof 23, which accommodates in its thickness at least one electrode 12, is the one with which the surgeon performs the dissection of the tissues with greater resistance. Said dissection by diathermy and the consequent coagulation are typically performed thanks to the diathermy induced by the Joule effect determined by the passage of monopolar current.

As shown in figure 2A, the contact portion 7 comprises a protruding portion to be directed towards the anatomical tissue to be separated with length dp. The length dp is preferably comprised between 40 mm and 60 mm and even more preferably is 50 mm. This protruding portion may be deformable.

The device 1 is such that it can be manoeuvred and managed by the surgeon responding to the variables needed for each individual case to be treated, taking into account first of all the diversity of the anatomical tissues that can be involved and that typically consist in offering a different resistance to dissection that may therefore require the alternation of the use of the technologies of hydro-dissection, as well as those of dissection induced by diathermy such as electro-dissection.

In order to implement the one and/or the other of the two techniques, the surgeon can act on the appropriate buttons 18 and 19 positioned on the actuation portion 17 of the device 1. In particular, the actuation portion 17 is preferably positioned near the second end 4, and even more preferably at a distance comprised between 40 and 60 mm from the point where the protruding portion of the contact portion 7 is lengthened. Preferably, the device according to this example is such that the protruding portion of the contact portion 7 has a thickness, understood as the sum of the thickness of the first sub-portion 8 shaped like a strip and the second sub-portion 9 shaped like a strip, comprised between 0.8 mm and 2.0 mm, preferably of 1.3 mm.

As mentioned above, the first sub-portion 8 has a greater extension, both in terms of width and length, than the second sub-portion 9 which rises on at least one of the surfaces thereof. More in detail, the first sub-portion 8 extends beyond the straight end of the second sub-portion 9 by at least 0.5 mm and preferably by at least 1 mm. In other words, the distance d between the first dissecting means 5 and the second dissecting means 6 (and/or the fluid dispensing means 11) is at least 0.5 mm and preferably at least 1 mm, in particular it is comprised between 2 mm and 3 mm.

The first sub-portion 8 shaped like a strip, as well as the tip of the contact portion 7 as a whole, has a width comprised between 2.0 mm and 4.0 mm and preferably of 3.0 mm.

As repeatedly stated, the peculiarity of the device 1 consists essentially in comprising in its structure the structural components necessary for the implementation of the dissection of tissues with specific resistance and, at the same time, not only in associating all the advantages derivable from the surgeon's choice of the dissection technique that best suits the case to be treated, but above all in allowing to enhance the effectiveness of both techniques with respect to the case in which they are implemented by means of distinct devices that as such separately perform hydro-dissection or electro-dissection, also overcoming all the disadvantages that can be found during the separate implementation of the methodologies in question. It follows that the particular structural configuration, as well as the same design of the device 1, and in particular of the profile and of the thought-out extension of the tip of the contact portion 7, unveil a real synergistic cooperation between its structural elements with the result of making the surgical procedure itself easier, improving its effectiveness, as well as increasing its temporal durability.

In this regard, a key role is played by the spatial configuration of the nozzles that emit the saline solution and $CO_2$. In fact, it can be noted that the possibility of emitting said substances from nozzles that are retracted with respect to the arcuate end 23 of the tip of the contact portion 7, is of crucial importance in order to have a substantial advantage in terms of increased possibility of performing the dissection of the tissues of interest with accurate precision. The $CO_2$ jet, typically emitted continuously, in addition to favouring the removal of any toxic smokes due to the electro-dissection of the tissues, frees the affected area from all the substances that can hinder the visibility of the field involved in the treatment, thus making the latter easier to perform. Preferably, for an effective operation of the liquid and gas jet, the point of emission of the saline aerosol and of the $CO_2$ gas is retracted by about 2.0-3.0 mm from the centre of the arcuate end 23 of the tip of the contact portion 7.

In one example, the second sub-portion 9 is sliding with respect to the first sub-portion 8 so that the distance d between the first dissecting means 5 and the second dissecting means 6 (but also from the fluid dispensing means 11) along the longitudinal direction L of the main body 2 is variable. In other words, the second sub-portion 9 is sliding and longitudinally movable on the body of the first sub-portion 8 shaped like a strip so that the surgeon can retract the emission of the aerosol (and/or of gas) at will by properly distancing it from the arcuate end 23 of the tip of the contact portion 7. It can be noted that the possibility of modulating the distance of the two dissecting systems thanks to the longitudinal sliding of the second sub-portion 9, allows to optimally focus the jet of the fluid so as to exert a greater or lesser mechanical force in dissecting the tissues with less resistance. A similar effect can also be obtained by varying (increasing or decreasing) the jet of the fluid exiting the first nozzle 13 of the fluid dispensing means 11 (e.g., $CO_2$) and/or the jet of the fluid exiting the second nozzle 14 of the second dissecting means 6 (e.g., saline). Of course, for the focusing effectiveness to be increased, the modulation of the distance of the dissecting systems by means of a mechanical sliding movement and the variation of the intensity of the fluid (gas or liquid) flow jet can be combined.

In all the embodiments thereof, the device 1 can be made of metallic and polymeric materials and/or composites, and with combinations thereof.

The device 1 according to the present invention is used in all areas of surgery and typically in cardiovascular surgery.

The device may further comprise an analysis module 21 having at least one light spectrum analyzer and one or more sensors for monitoring in real time the electrical and thermodynamic behaviour of the device 1. The sensor system and a microcontroller automatically adjust the device 1, recognizing the type of tissue and the state thereof based on the spectrum analysis of the brightness of the arc produced.

Advantageously, the sensor system and the digital microcontroller are adapted to automatically adjust the operation of the device in real time during use, in order to use only the energy that is actually necessary and avoiding potential undesirable effects. A system based on machine learning technology can be used for this purpose.

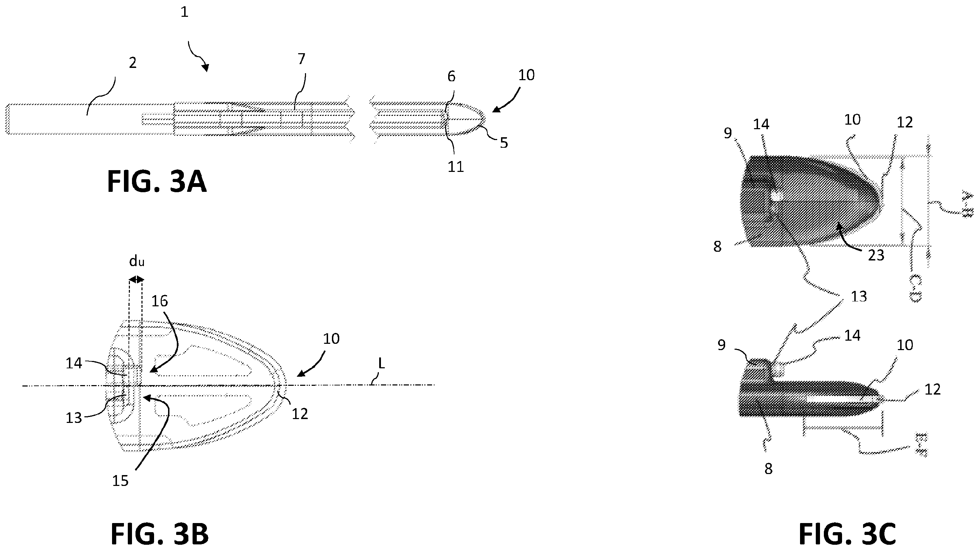

Figures 3A-3C show a second embodiment of the device 1. In this case, the first outlet orifice 15 is retracted and spaced from the second outlet orifice 16 along the longitudinal direction L of the main body 2 at a distance $d_u$. In other words, the fluid jet (e.g. gas) exiting from the fluid dispensing means 11 and the fluid jet (e.g. liquid) exiting from the second dissecting means 6 exit from two different points, staggered to each other by a length $d_u$, along the main body 2, i.e. along the contact portion 7 of the device 1. This is particularly advantageous when use is to be made of hydro-dissection by dispensing fluid (e.g., liquid, saline) from the second nozzle 14 of the second dissecting means 6 so as to have an adequate spacing of the fluid jet (e.g., gas, $CO_2$) emitted from the first nozzle 13 of the fluid dispensing means 11. In addition, the staggering of the two orifices serves to determine a convergence of the two fluid flows (e.g. gas and liquid) such that the mechanical jet is focused exactly in front of the electrode 12. Of course, the pressure of the gas and liquid changes the mechanical force at the level of the convergence of the jet. Thus, the staggering length $d_u$ is chosen such that two fluid flows with different density, i.e. liquid and gas, can converge on the same region in front of the electrode 12. In particular, the distance between the first outlet orifice 15 for the emission of the fluid from the first nozzle 13 (e.g., gas, CO2) and the electrode 12 is greater than the distance between the second outlet orifice 16 for the emission of the fluid from the second nozzle 14 (e.g., liquid, saline solution) and the electrode 12. Advantageously, the distance $d_u$ is variable. In other words, the first nozzle 13 and/or the second nozzle 14 may be sliding along the longitudinal direction L of the main body 2. In this way, it is possible to choose the most suitable distance $d_u$ depending on the density of the fluids emitted by the first nozzle 13 and by the second nozzle 14. For example, $d_u$ can be comprised between 0 mm and 1 mm.

In particular, figure 3A shows a detailed view of the structural diagram of the device 1 and in particular of the tip thereof of the contact portion 7. Figure 3B illustrates a magnification of the tip observable in figure 3A. From this figure it can be seen that the second orifice 16 of the second nozzle 14 for the emission of the saline solution is spaced by a length $d_u$ from the first orifice 15 of the first nozzle 13 for the emission of the $CO_2$.

Figure 3C shows a plan view and a side view of the tip highlighting the structural components of the latter also in relation to their dimensions.

More in detail, figure 3C shows the width of the electrode 12 emerging from the arcuate end 23 of the tip of the contact portion 7 along the straight line AB, along the straight line CD and along the straight line EF. By way of non-limiting example, these dimensions respectively assume a value comprised between 2.5 and 2.7 mm, preferably 2.59 mm, a value comprised between 2.4 and 2.6 mm, preferably 2.51 mm, and a value comprised between 2.2 and 2.4 mm, preferably 2.28 mm.

To the surgical device described above, a person skilled in the art, in order to meet further and contingent needs, may make numerous further modifications and variations, all of which are included in the scope of protection of the present invention as defined by the appended claims.

## Claims

1.   Surgical device (1) for dissecting an anatomical tissue comprising:

a main body (2) extending along a longitudinal direction (L) and having a first end (3) and a second end (4) comprising a contact portion (7) adapted to come into contact with the anatomical tissue, in particular having a spatula shape, wherein the contact portion (7) comprises a first sub-portion (8) and a second sub-portion (9) extending over the first sub-portion (8);
at least a first dissecting means (5) positioned on the first sub-portion (8); and

at least a second dissecting means (6) positioned on the second sub-portion (9),

wherein the first dissecting means (5) is disposed on an end region (10) of the second end (4) and the second dissecting means (6) is retracted and spaced from the first dissecting means (5) along the longitudinal direction (L) of the main body (2) at a distance d,

**characterised by**

further comprising a fluid dispensing means (11) positioned on the second sub-portion (9) and side-by-side to the second dissecting means (6), said fluid dispensing means (11) being connectable to a fluid reservoir through a connector at the first end (3).

2.  Device (1) according to claim 1, wherein the fluid dispensing means (11) comprises at least a first nozzle (13) for dispensing a pressurized fluid, in particular for dispensing carbon dioxide.

3.  Device (1) according to one of the preceding claims, wherein the second dissecting means (6) comprises at least a second nozzle (14) for dispensing a pressurized fluid, in particular for dispensing a saline solution, for a hydro-dissection of the anatomical tissue, said second dissecting means (6) being connectable to a fluid reservoir through a connector at the first end (3).

4.  Device (1) according to claim 2 and 3, wherein the first nozzle (13) comprises a first outlet orifice (15) and the second nozzle (14) comprises a second outlet orifice (16), the first nozzle (13) being side-by-side to the second nozzle (14), and wherein the first outlet orifice (15) and the second outlet orifice (16) are arranged on the same plane orthogonal to the longitudinal direction (L) of the main body (2).

5.  Device (1) according to claim 2 and 3, wherein the first nozzle (13) comprises a first outlet orifice (15) and the second nozzle (14) comprises a second outlet orifice (16), the first nozzle (13) being side-by-side to the second nozzle (14), and wherein the first outlet orifice (15) is retracted and spaced from the second outlet orifice (16) along the longitudinal direction (L) of the main body (2) at a distance du.

6.  Device (1) according to claim 5, wherein the distance between the first outlet orifice (15) for the emission of fluid from the first nozzle (13) and the electrode (12) is greater than the distance between the second outlet orifice (16) for the emission of fluid from the second nozzle (14) and the electrode (12).

7.  Device (1) according to one of claims 4 to 6, wherein the first nozzle (13) and/or the second nozzle (14) are sliding along the longitudinal direction (L) of the main body (2).

8.  Device (1) according to one of the preceding claims, wherein the first dissecting means (5) comprises:

    a. an electrode (12) for an electro-dissection of the anatomical tissue, said electrode (12) being connectable to a current generator via a connector at the first end (3), and/or
    b. an ultrasound emitter for dissection by ultrasound induction of the anatomical tissue, said emitter being connectable to a current generator through a connector at the first end (3).

9.  Device (1) according to one of the preceding claims, wherein the second sub-portion (9) is sliding with respect to the first sub-portion (8) so that the distance d between the second dissecting means (6) and the first dissecting means (5) along the longitudinal direction (L) of the main body (2) is variable.

10. Device (1) according to one of the preceding claims, further comprising an actuation portion (17) arranged on the main body (2) and having a first button (18) for activating the first dissecting means (5) and a second button (19) for activating the second dissecting means (6).

11. Device (1) according to one of the preceding claims, further comprising a flow regulator (20) arranged on the main body (2) for varying the flow of fluid dispensed by the fluid dispensing means (11), wherein in particular the fluid flow is variable between 0 l/min and 1 l/min.

12. Device (1) according to one of the preceding claims, further comprising an analysis module (21) having at least one light spectrum analyzer and one or more sensors for monitoring in real time the electrical and thermodynamic behavior of the device (1).

13. Device (1) according to one of the preceding claims, wherein the contact portion (7) is removably attachable to the main body (2) at the second end (4).

14. Device according to one of the preceding claims, wherein the contact portion 7 is deformable and comprises a protruding portion to be directed towards the anatomical tissue to be separated, said protruding portion having a length dp between 40 mm and 60 mm, in particular of 50 mm.

15. Device (1) according to one of the preceding claims, wherein the distance d between the first dissecting means 5 and the second dissecting means 6 and/or the fluid dispensing means 11 is at least 0.5 mm and preferably at least 1 mm, in particular it is comprised between 2 mm and 3 mm.

**Patentansprüche**

1. Chirurgische Vorrichtung (1) zum Präparieren von anatomischem Gewebe, umfassend:

   einen Hauptkörper (2), der sich in einer Längsrichtung (L) erstreckt und ein erstes Ende (3) sowie ein zweites Ende (4) aufweist, das einen Kontaktabschnitt (7) umfasst, der angepasst ist, um mit dem anatomischen Gewebe in Kontakt zu kommen, und insbesondere eine spatelförmige Gestalt aufweist, wobei der Kontaktabschnitt (7) einen ersten Teilabschnitt (8) und einen zweiten Teilabschnitt (9) umfasst, der sich über den ersten Teilabschnitt (8) erstreckt;
   mindestens eine erste Präpariereinrichtung (5), die auf dem ersten Teilabschnitt (8) angeordnet ist;
   und mindestens eine zweite Präpariereinrichtung (6), die auf dem zweiten Teilabschnitt (9) angeordnet ist, wobei die erste Präpariereinrichtung (5) in einem Endbereich (10) des zweiten Endes (4) angeordnet ist und die zweite Präpariereinrichtung (6) von der ersten Präpariereinrichtung (5) in der Längsrichtung (L) des Hauptkörpers (2) in einem Abstand d zurückgesetzt und beabstandet ist,
   **dadurch gekennzeichnet,**
   **dass** sie ferner eine Fluidabgabeeinrichtung (11) umfasst, die auf dem zweiten Teilabschnitt (9) und Seite an Seite mit der zweiten Präpariereinrichtung (6) angeordnet ist, wobei die Fluidabgabeeinrichtung (11) über ein Verbindungsstück an dem ersten Ende (3) mit einem Fluidreservoir verbunden werden kann.

2. Vorrichtung (1) nach Anspruch 1, wobei die Fluidabgabeeinrichtung (11) mindestens eine erste Düse (13) zum Abgeben eines unter Druck stehenden Fluids, insbesondere zum Abgeben von Kohlendioxid, umfasst.

3. Vorrichtung (1) nach einem der vorhergehenden Ansprüche, wobei die zweite Präpariereinrichtung (6) mindestens eine zweite Düse (14) zum Abgeben eines unter Druck stehenden Fluids, insbesondere zum Abgeben einer Kochsalzlösung, für eine Hydropräparation des anatomischen Gewebes umfasst, wobei die zweite Präpariereinrichtung (6) über ein Verbindungsstück an dem ersten Ende (3) mit einem Fluidreservoir verbunden werden kann.

4. Vorrichtung (1) nach Anspruch 2 und 3, wobei die erste Düse (13) eine erste Auslassöffnung (15) umfasst und die zweite Düse (14) eine zweite Auslassöffnung (16) umfasst, wobei die erste Düse (13) Seite an Seite mit der zweiten Düse (14) angeordnet ist und die erste Auslassöffnung (15) und die zweite Auslassöffnung (16) in derselben Ebene orthogonal zu der Längsrichtung (L) des Hauptkörpers (2) angeordnet sind.

5. Vorrichtung (1) nach Anspruch 2 und 3, wobei die erste Düse (13) eine erste Auslassöffnung (15) umfasst und die zweite Düse (14) eine zweite Auslassöffnung (16) umfasst, wobei die erste Düse (13) Seite an Seite mit der zweiten Düse (14) angeordnet ist und die erste Auslassöffnung (15) von der zweiten Auslassöffnung (16) in der Längsrichtung (L) des Hauptkörpers (2) in einem Abstand du zurückgesetzt und beabstandet ist.

6. Vorrichtung (1) nach Anspruch 5, wobei der Abstand zwischen der ersten Auslassöffnung (15) für den Austritt von Fluid aus der ersten Düse (13) und der Elektrode (12) größer als der Abstand zwischen der zweiten Auslassöffnung (16) für den Austritt von Fluid aus der zweiten Düse (14) und der Elektrode (12) ist.

7. Vorrichtung (1) nach einem der Ansprüche 4 bis 6, wobei die erste Düse (13) und/oder die zweite Düse (14) entlang der Längsrichtung (L) des Hauptkörpers (2) gleiten.

8. Vorrichtung (1) nach einem der vorhergehenden Ansprüche, wobei die erste Präpariereinrichtung (5) Folgendes umfasst:

a. eine Elektrode (12) für eine Elektro-Präparation des anatomischen Gewebes, wobei die Elektrode (12) über ein Verbindungsstück an dem ersten Ende (3) mit einem Stromgenerator verbunden werden kann, und/oder

b. einen Ultraschallemitter zur Präparation des anatomischen Gewebes durch Ultraschallinduktion, wobei der Emitter über ein Verbindungsstück an dem ersten Ende (3) mit einem Stromgenerator verbunden werden kann.

9. Vorrichtung (1) nach einem der vorhergehenden Ansprüche, wobei der zweite Teilabschnitt (9) relativ zu dem ersten Teilabschnitt (8) gleitet, derart, dass der Abstand d zwischen der zweiten Präpariereinrichtung (6) und der ersten Präpariereinrichtung (5) entlang der Längsrichtung (L) des Hauptkörpers (2) variabel ist.

10. Vorrichtung (1) nach einem der vorhergehenden Ansprüche, ferner umfassend einen auf dem Hauptkörper (2) angeordneten Betätigungsabschnitt (17) mit einer ersten Taste (18) zum Aktivieren der ersten Präpariereinrichtung (5) und einer zweiten Taste (19) zum Aktivieren der zweiten Präpariereinrichtung (6).

11. Vorrichtung (1) nach einem der vorhergehenden Ansprüche, ferner umfassend einen auf dem Hauptkörper (2) angeordneten Flussregler (20) zum Variieren des von der Fluidabgabeeinrichtung (11) abgegebenen Fluidflusses, wobei der Fluidfluss insbesondere zwischen 0 l/min und 1 l/min variabel ist.

12. Vorrichtung (1) nach einem der vorhergehenden Ansprüche, ferner umfassend ein Analysemodul (21) mit mindestens einem Lichtspektrumanalysator und einem oder mehreren Sensoren zum Überwachen des elektrischen und thermodynamischen Verhaltens der Vorrichtung (1) in Echtzeit.

13. Vorrichtung (1) nach einem der vorhergehenden Ansprüche, wobei der Kontaktabschnitt (7) an dem zweiten Ende (4) abnehmbar an dem Hauptkörper (2) befestigt werden kann.

14. Vorrichtung nach einem der vorhergehenden Ansprüche, wobei der Kontaktabschnitt 7 verformbar ist und einen hervorstehenden Abschnitt umfasst, der auf das zu trennende anatomische Gewebe zu richten ist, wobei der hervorstehende Abschnitt eine Länge dp zwischen 40 mm und 60 mm, insbesondere von 50 mm, aufweist.

15. Vorrichtung (1) nach einem der vorhergehenden Ansprüche, wobei der Abstand d zwischen der ersten Präpariereinrichtung 5 und der zweiten Präpariereinrichtung 6 und/oder der Fluidabgabeeinrichtung 11 mindestens 0,5 mm und vorzugsweise mindestens 1 mm beträgt, insbesondere zwischen 2 mm und 3 mm liegt.

**Revendications**

1. Dispositif chirurgical (1) pour disséquer un tissu anatomique comprenant :

un corps principal (2) s'étendant le long d'une direction longitudinale (L) et ayant une première extrémité (3) et une seconde extrémité (4) comprenant une portion de contact (7) adaptée pour venir en contact avec le tissu anatomique, en particulier ayant une forme de spatule, la portion de contact (7) comprenant une première sous-portion (8) et une seconde sous-portion (9) s'étendant sur la première sous-portion (8) ;
au moins un premier moyen de dissection (5) positionné sur la première sous-portion (8) ; et
au moins un second moyen de dissection (6) positionné sur la seconde sous-portion (9),
le premier moyen de dissection (5) étant disposé sur une zone d'extrémité (10) de la seconde extrémité (4) et le second moyen de dissection (6) étant rétracté et espacé du premier moyen de dissection (5) le long de la direction longitudinale (L) du corps principal (2) à une distance d,
**caractérisé par**
comprenant en outre un moyen de distribution de fluide (11) positionné sur la seconde sous-portion (9) et côte-à-côte au second moyen de dissection (6), ledit moyen de distribution de fluide (11) étant connectable à un réservoir de fluide par un connecteur à la première extrémité (3).

2. Dispositif (1) selon la revendication 1, dans lequel le moyen de distribution de fluide (11) comprend au moins une première buse (13) pour distribuer un fluide pressurisé, en particulier pour distribuer du dioxyde de carbone.

3. Dispositif (1) selon l'une des revendications précédentes, dans lequel le second moyen de dissection (6) comprend au moins une seconde buse (14) pour distribuer un fluide pressurisé, en particulier pour distribuer une solution saline, pour une hydro-dissection du tissu anatomique, ledit second moyen de distribution (6) étant connectable à un réservoir de fluide par un connecteur à la première extrémité (3).

4. Dispositif (1) selon la revendication 2 et 3, dans lequel la première buse (13) comprend un premier orifice de sortie (15) et la seconde buse (14) comprend un second orifice de sortie (16), la première buse (13) étant côte-à-côte à la seconde buse (14) et le premier orifice de sortie (15) et le second orifice de sortie (16) étant disposés sur le même plan orthogonal à la direction longitudinale (L) du corps principal (2).

5. Dispositif (1) selon la revendication 2 et 3, dans lequel la première buse (13) comprend un premier orifice de sortie (15) et la seconde buse (14) comprend un second orifice de sortie (16), la première buse (13) étant côte-à-côte à la seconde buse (14) et le premier orifice de sortie (15) étant rétracté et espacé du second orifice de sortie (16) le long de la direction longitudinale (L) du corps principal (2) à une distance du.

6. Dispositif (1) selon la revendication 5, dans lequel la distance entre le premier orifice de sortie (15) pour l'émission de fluide de la première buse (13) et l'électrode (12) est plus grande que la distance entre le second orifice de sortie (16) pour l'émission de fluide de la seconde buse (14) et l'électrode (12).

7. Dispositif (1) selon l'une des revendications 4 à 6, dans lequel la première buse (13) et/ou la seconde buse (14) sont coulissantes le long de la direction longitudinale (L) du corps principal (2).

8. Dispositif (1) selon l'une des revendications précédentes, dans lequel le premier moyen de dissection (5) comprend :

   a. une électrode (12) pour une électro-dissection du tissu anatomique, ladite électrode (12) étant connectable à un générateur de courant via un connecteur à la première extrémité (3) et/ou
   b. un émetteur d'ultrasons pour la dissection par induction d'ultrasons du tissu anatomique, ledit émetteur étant connectable à un générateur de courant par un connecteur à la première extrémité (3).

9. Dispositif (1) selon l'une des revendications précédentes, dans lequel le seconde sous-portion (9) est coulissante par rapport à la première sous-portion (8) de sorte que la distance d entre le second moyen de dissection (6) et le premier moyen de dissection (5) le long de la direction longitudinale (L) du corps principal (2) est variable.

10. Dispositif (1) selon l'une des revendications précédentes, comprenant en outre une portion d'actionnement (17) disposée sur le corps principal (2) et ayant un premier bouton (18) pour activer le premier moyen de dissection (5) et un second bouton (19) pour activer le second moyen de dissection (6).

11. Dispositif (1) selon l'une des revendications précédentes, comprenant en outre un régulateur d'écoulement (20) disposé sur le corps principal (2) pour faire varier l'écoulement de fluide distribué par le moyen de distribution de fluide (11), en particulier l'écoulement de fluide étant variable entre 0 l/min et 1 1/min.

12. Dispositif (1) selon l'une des revendications précédentes, comprenant en outre un module d'analyse (21) ayant au moins un analyseur de spectre de lumière et un ou plusieurs capteurs pour suivre en temps réel le comportement électrique et thermodynamique du dispositif (1).

13. Dispositif (1) selon l'une des revendications précédentes, dans lequel la portion de contact (7) est attachable de manière amovible au corps principal (2) à la seconde extrémité (4).

14. Dispositif selon l'une des revendications précédentes, dans lequel la portion de contact 7 est déformable et comprend une portion en saillie à diriger vers le tissu anatomique pour être séparé, ladite portion en saillie ayant une longueur dp comprise entre 40 mm et 60 mm, en particulier de 50 mm.

15. Dispositif (1) selon l'une des revendications précédentes, dans lequel la distance de entre le premier moyen de dissection 5 et le second moyen de dissection 6 et/ou le moyen de distribution de fluide 11 est d'au moins 0,5 mm et de préférence d'au moins 1 mm, en particulier elle est comprise entre 2 mm et 3 mm.

**FIG. 1**

EP 4 398 822 B1

**FIG. 2A**

**FIG. 2B**

**FIG. 2C**

EP 4 398 822 B1

FIG. 3A

FIG. 3B

FIG. 3C

EP 4 398 822 B1

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2020093537 A1
- WO 03096871 A2